# EUROPÄISCHE PATENTSCHRIFT

(11) **EP 2 142 626 B1**
(45) Veröffentlichungstag und Bekanntmachung des Hinweises auf die Patenterteilung: **04.12.2013**
(21) Anmeldenummer: 07866275.6
(22) Anmeldetag: 31.12.2007
(51) Int. Cl.: C11D 3/00, C11D 3/20, C11D 3/39, C11D 7/26, A61L 12/12

(54) **KONTAKTLINSENREINIGER**
CONTACT LENS CLEANER
AGENT POUR LE NETTOYAGE DE LENTILLES DE CONTACT

(30) Priorität: 29.12.2006 DE 102006062616
(43) Veröffentlichungstag der Anmeldung: 13.01.2010
(73) Patentinhaber: NEV'S New Ecological Vision'S GmbH, 59227 Ahlen (DE)
(72) Erfinder: REICHWAGEN, Sven, 59227 Ahlen (DE)
(74) Vertreter: Isfort, Olaf
(86) Internationale Anmeldenummer: PCT/EP2007/011475
(87) Internationale Veröffentlichungsnummer: WO 2008/080627

(56) Entgegenhaltungen:
- EP-A- 0 487 994
- GB-A- 1 577 396
- GB-A- 2 003 033
- US-A- 4 863 627
- US-A- 4 986 963
- DATABASE WPI Week 200415 Derwent Publications Ltd., London, GB; AN 2004-149048 XP002479928 & JP 2004 024715 A (LION CORP) 29. Januar 2004 (2004-01-29)
- DATABASE WPI Week 200257 Derwent Publications Ltd., London, GB; AN 2002-532006 XP002479929 & JP 2002 129197 A (SHIKOKU KASEI KOGYO KK) 9. Mai 2002 (2002-05-09)
- DATABASE WPI Week 198831 Derwent Publications Ltd., London, GB; AN 1988-215599 XP002479930 & JP 63 150394 A (TOYO KAGAKU KENKYUS) 23. Juni 1988 (1988-06-23)

## Beschreibung

Die Erfindung betrifft einen Kontaktlinsenreiniger in Tablettenform.

Die Reinigung von Kontaktlinsen ist ein für Kontaktlinsenträger essentielles Gebot. Eine unzureichende Reinigung von Kontaktlinsen kann zu schweren Augeninfektionen führen. Die Reinigungsflüssigkeiten selbst können, wenn sie ins Auge gelangen, dort zu Schäden führen. Des Weiteren können die Reinigungsflüssigkeiten beim Anwender Hautirritationen, insbesondere an den Händen, herbeiführen.

Herkömmliche Reinigungsflüssigkeiten enthalten vielfach Wasserstoffperoxid, das sowohl für die desinfizierende als auch für die Reinigungswirkung verantwortlich zeichnet. Zusätzlich zur Desinfektionswirkung zeigt H₂O₂ auch eine starke Reinigungswirkung durch die Generierung von aktivem Sauerstoff.

Eine Vielzahl von Mikroorganismen ist allerdings durch Katalasen gegen die Wirkung von H₂O₂ mehr oder weniger geschützt. Solche Mikroorganismen können zwar immer noch durch H₂O₂ im Wachstum gehemmt oder abgetötet werden, jedoch sind hierzu recht hohe Konzentrationen erforderlich. Derartige Konzentrationen wirken dann aber stark gewebereizend, so dass ein Kontakt mit den Augen und ggf. auch der Haut des Anwenders strikt vermieden werden muß. Dies ist aber nicht immer zuverlässig möglich, weshalb H₂O₂-Konzentrationen häufig für eine Desinfektion zu niedrig eingestellt werden.

Die EP 0 487 994 A1 beschreibt einen Kontaktlinsenreiniger in Tablettenform, der sich aus einer Peroxidkomponente und einer Desaktivierungskomponente zusammensetzt. Die Peroxidkomponente enthält eine anorganische oder eine organische Peroxidverbindung, die Desaktivierungskomponente ein Schwermetallsalz und Zitronensäure, die verzögert freigesetzt werden.

Die Probleme bei der Linsenhygiene, die insbesondere weiche Kontaktlinsen betreffen, haben viel zur Verbreitung der Tages- und Monatslinsen beigetragen. Die begrenzte Haltbarkeit bzw. Verwendungsdauer dieser Linsen vermindert die hygienischen Probleme dadurch, dass die Linsen ausgemustert werden, bevor es zu einer problematischen Akkumulation von mikrobiellen Keimen auf der Oberfläche kommen kann. Allerdings sind diese Tages- und Monatslinsen ein relativ aufwendiger Ersatz für Dauerlinsen, insbesondere was die Kosten anbetrifft.

Angesichts dessen besteht ein Bedarf an einem Kontaktlinsenreiniger, der der mikrobiellen Kontamination von Kontaktlinsen zuverlässig entgegenwirkt, durch einfaches Abspülen beseitigt werden kann und Augen- und Hautreizungen zuverlässig vermeidet.

Diese Aufgabe wird mit dem erfindungsgemäßen Kontaktlinsenreiniger gemäß Anspruch 1 gelöst.

Es wurde gefunden, dass Perzitronensaüre in außerordentlich niedriger Konzentration stark mikrobizid wirkt. Mikrobizid in diesem Zusammenhang bedeutet, dass sie Bakterien, Viren, Pilze und andere niedere Lebewesen zuverlässig abtöten. Dabei reicht es aus, dass der erfindungsgemäße Kontaktlinsenreiniger in wässeriger Lösung in einer Konzentration von 0,001 bis 5 Gew.-% vorliegt, vorzugsweise 0,01 bis 1 Gew.-% und insbesondere in einer Konzentration von 0,05 bis 0,5 Gew.-% vorhanden ist.

Der erfindungsgemäße Kontaktlinsenreiniger kann nach dem Einsatz ohne Weiteres durch den Abfluss entsorgt werden. Es reicht aus, die Kontaktlinsen gründlich mit Wasser nachzuspülen. Mit dem Reiniger in Kontakt gekommene Hautoberflächen können mit Wasser abgespült werden.

Die H₂O₂ generierende Verbindung ist ein Peroxosulfat, Peroxodisulfat, Percarbonat, oder Harnstoffperoxid. Hier sind insbesondere die Salze der Peroxover bindungen vorteilhaft, sobald eine Salzform generiert werden kann, beispielsweise die Alkali- oder Ammoniumsalze der Peroxoschwefelsäure bzw. Peroxodischwefelsäure. Bei Percarbonat handelt es sich um Natriumcarbonat, das Wasserstoffperoxid als Kristallwasser angelagert enthält. Besonders bevorzugt sind die anorganischen Peroxosalze, und hiervon Kaliumcaroat, 2 KHSO₅ x KHSO₄ x K₂SO₄.

Die Verwendung von Zitronensäure, ihrer Salze oder Anhydride und der H₂O₂ generierenden Verbindung gestattet es, den Kontaktlinsenreiniger in Tablettenform herzustellen. Salze der H₂O₂ generierenden Verbindungen liegen ohnehin in fester Form vor und sind in fester Form auch beständig gegen Zerfall. Eine diese Mittel enthaltende Tablette enthält Tenside und kann übliche Träger- und Hilfsstoffe enthalten und beispielsweise in einer Blisterpackung vorliegen.

Die H₂O₂ generierende Verbindung liegt in einem 2 bis 6-fachen molaren Überschuss auf die organische Säure, ihr Salz oder ihr Anhydrid vor. Diese Maßnahme stellt sicher, dass auch nach Umsetzung der Säure zur Peroxycarbonsäure Peroxid verbleibt, das zur Generierung von aktivem Sauerstoff dient und die Reinigungswirkung fördert. Bevorzugt ist insbesondere ein 4 bis 5-facher Überschuss an H₂O₂ generierender Verbindung.

Soweit diese Variante des Kontaktlinsenreinigers in Tablettenform vorliegt, liegen die beiden Komponenten in der Tablette nebeneinander in stabiler Form vor. In wässerige Lösung gebracht entstehen allenfalls geringe Mengen der organischen Säure, so dass sich ein pH-Wert einstellt, der nicht im stark sauren Bereich liegt. Tatsächlich können Formulierungen herbeigeführt werden, die im Wesentlichen neutral sind. Solche Formulierungen sind erfindungsgemäß bevorzugt, da sie zu einem relativ raschen Zerfall der Peroxybestandteile der erfindungsgemäß hergestellten wässerigen Reinigungslösung führt. Bei beispielsweise der Verwendung von Zitronensäure und Kaliumcaroat als Bestandteilen einer Tablette liegt bei Beendigung des Reinigungsvorganges in der Reinigungslösung praktisch nur noch Zitronensäure und Kaliumsulfat vor, die bedenkenlos entsorgt werden können.

Die Tabletten können Tablettierungshilfmittel und dergleichen in üblichen Mengen enthalten. Die Tabletten enthalten ferner bis zu 10 Gew.-% eines Essigsäuresalzes enthalten, vorzugsweise Natriumacetat. Natriumacetat trägt zur Reinigungswirkung der Lösung bei und kann die Auflösung der Peroxycarbonsäure verbessern.

Die Tablette sollte die notwendigen Bestandteile, d. h. die Zitronensäure bzw. ihre Derivate und die H₂O₂ generierende Verbindung in einer Menge enthalten, die in der zur Anwendung kommenden Reinigungslösung eine Konzentration an Peroxycarbonsäure im Bereich von 0,001 bis 5 Gew.-% erzeugt, bevorzugt 0,01 bis 1 Gew.-% und insbesondere 0,05 bis 0,5 Gew.-%.

Die Tabletten können schließlich im Kit mit einem Deaktivierungsmittel vorliegen, etwa einer Pyruvat- oder Thiosulfattinktur oder -tablette oder einem Platindrahtsieb oder einer solchen Scheibe.

### Testbedingungen nach DIN

### 1. Allgemeines

- Testverfahren nach DIN EN ISO 14729 "Augenoptik-Kontaktlinsenpflegemittel-Mikrobiologische Anforderungen und Prüfverfahren für Produkte und System zum Hygienemanagement von Kontaktlinsen";
- Testkeime:

| | |
|---|---|
| Pseudomonas aeruginosa | ATCC 9027 |
| Staphylococcus aureus | ATCC 6538 |
| Serratia marcescens | ATCC 13880 |
| Candida albicans | ATCC 10231 |
| Fusarium solani | ATCC 36031 |

- Verdünnungsmedium: isotonische Kochsalzlsg.; ersatzweise Mineralwasser, Leitungswasser
- Konzentration in der Anwendungslösung Reiniger

| | |
|---|---|
| 0.001 % - 7 % | Flüssiger |
| 0.001 % - 13 % | Feststoff Reiniger |

- Inaktivatorenauswahl:
Natriumthiosulfat oder Pyruvat als Kurz- oder Langzeitinaktivierungsmittel
Platindisk als Langzeitinaktivierungsmittel

- Puffer: Phosphatpuffer (NaH₂PO₄, Na₂HPO₄, Na₃PO₄) eingestellt auf einem pH-Wert im neutralen Bereich

### Beispiel 1: (Referenz)

50 g Zitronensäure und 352 g Kaliumperoxodisulfat in Pulverform werden miteinander gemischt und zu Tabletten à 2 g verpresst.

Eine Tablette, aufgelöst in 100 ml Leitungswasser, ergibt eine etwa 0,25 %ige Lösung von Peroxyzitronensäure. Der Überschuss an Kaliumperoxodisulfat (5 Mol Kaliumperoxodisulfat pro Mol Zitronensäure) führt zu einer langsam einsetzenden, länger anhaltenden Sauerstoffentwicklung. Der Reinigungsprozess ist nach wenigen Minuten abgeschlossen.

### Testversuche:

Mit den Mikroorganismen S. aureus ATTC 6538, E. coli NCTC 10538, P. merabilis ATCC 14153, P. aeruginosa ATCC 15442, E. hirae ATCC 10541 und C. albicans ATCC 10231 (jeweils 2 bis 5 x 10⁹ KbE) ergab sich bei einer Konzentration der Peroxycarbonsäure von 0,5 % eine Inaktivierung innerhalb von 5 min.

## Patentansprüche

1. Kontaktlinsenreiniger in Tablettenform, enthaltend Zitronensäure, ihr Salz oder ihr Anhydrid zusammen mit einer H₂O₂ generierenden Verbindung, ausgewählt aus Peroxosulfat, Peroxodisulfat, Percarbonat oder Harnstoffperoxid in einem molaren Überschuss von 2 bis 6 über die Zitronensäure, ihr Salz oder ihr Anhydrid, bis zu 10 Gew.-% eines Essigsäuresalzes sowie ein Tensid.

2. Kontaktlinsenreiniger nach Anspruch 1, **dadurch gekennzeichnet, dass** die H₂O₂ generierende Verbindung in einem 4- bis 5-fachen molaren Überschuss über die Zitronensäure, ihr Salz oder Anhydrid vorliegt.

## Claims

1. Contact lens cleaning agent in form of a tablet, comprising citric acid, its salt or its anhydride in combination with a H₂O₂ generating compound selected from peroxosulfate, peroxodisulfate, percarbonate or urea peroxide in a molar excess of 2 to 6 over citric acid, its salt or its anhydride, up to 10 weight % of an acetic acid salt, on a tenside.

2. Contact lens cleaner according to claim 1, **characterized in that** it comprises the H₂O₂ generating compound in a molar excess of 4 to 5 over citric acid, its salt or its anhydride.

## Revendications

1. Produit nettoyant pour lentilles de contact sous forme de comprimés, contenant de l'acide citrique, son sel ou son anhydride associés à un composé générant du H₂O₂, choisi à partir de péroxosulfate, de péroxodisulfate, de percarbonate ou de péroxide de carbamide dans un excédent molaire de 2 à 6 audessus de l'acide citrique, son sel ou son anhydride, jusqu'à 10 % du poids d'un sel d'acide acétique ainsi qu'un tenside.

2. Produit nettoyant pour lentilles de contact selon la revendication 1, **caractérisé par le fait que** le composé générant du H₂O₂ est présent dans un excédent molaire à multiplicateur de 4 à 5 au dessus de l'acide citrique, son sel ou son anhydride.
